# EUROPÄISCHE PATENTSCHRIFT

(11) **EP 0 668 725 B1**
(45) Veröffentlichungstag und Bekanntmachung des Hinweises auf die Patenterteilung: **15.01.1997**
(21) Anmeldenummer: 94900124.2
(22) Anmeldetag: 10.11.1993
(51) Int. Cl.: A23J 1/10, A23J 3/32, A23J 3/34

(54) **VERFAHREN ZUR HERSTELLUNG CHROMARMER PROTEINHYDROLYSATE**
METHOD OF PREPARING PROTEIN HYDROLYSATES LOW IN CHROMIUM
PROCEDE DE PREPARATION D'HYDROLYSATS PROTEIQUES A FAIBLE TENEUR EN CHROME

(30) Priorität: 19.11.1992 DE 4238979
(43) Veröffentlichungstag der Anmeldung: 30.08.1995
(73) Patentinhaber: Grünau Illertissen GmbH, D-89251 Illertissen (DE)
(72) Erfinder: EILERS, Eberhard, D-89081 Ulm (DE); SANDER, Andreas, D-89257 Illertissen (DE)
(74) Vertreter: Wilk, Hans-Christof, Dr.
(86) Internationale Anmeldenummer: EP9303151
(87) Internationale Veröffentlichungsnummer: WO9410856

(56) Entgegenhaltungen:
- DE-C- 972 090
- GB-A- 1 041 717
- DATABASE WPI Week 9129, Derwent Publications Ltd., London, GB; AN 91-209003 & DD,A,287 274 (BERLIN CHEMIE VEB) 21. Februar 1991
- DATABASE WPI Week 9131, Derwent Publications Ltd., London, GB; AN 91-225297 & HU,A,55 796 (PETI NITROGENMUVEK) 28. Juni 1991
- DATABASE WPI Week 8501, Derwent Publications Ltd., London, GB; AN 85-000519 & DD,A,212 983 (TECHN. MIKROBIOL.) 29. August 1984

## Beschreibung

### Gebiet der Erfidung

Die Erfindung betrifft ein Verfahren zur Herstellung chromarmer Proteinhydrolysate.

### Stand der Technik

Der Verbleib von Chromfalzspänen, die als Abfallprodukte bei der Herstellung chromgegerbter Leder anfallen, stellt in zunehmendem Maße ein Problem für die Lederindustrie dar. Der Grund dafür besteht darin, daß der Verbleib des Chromabfalls in Deponien die Gefahr einer Kontamination des Grundwassers durch Cr(III) und - nach einer eventuellen Oxidation - auch durch Cr(VI) in sich birgt. Nach Angaben von Taylor et al. fallen allein in den USA jährlich 54,000 Tonnen an Chromfalzspänen an ( J. Am. Leather Chem. Assoc. 1990, 85, 264).

Zu den ältesten Techniken des Entgerbens von Chromlederabfällen gehört das Kochen mit Alkali, meist Kalk- oder Magnesiumoxid. Exemplarisch sei in diesem Zusammenhang auf die US-Patentschrift 4 100 154 verwiesen. Nach der Lehre dieser Patentschrift wird die alkalische Hydrolyse von Chromleder bei Temperaturen oberhalb von ca. 90 °C in Gegenwart von Calciumoxid bzw. Calciumhydroxid durchgeführt. Durch Erhöhung der Reaktionstemperatur, die sich z. B. durch das Arbeiten in einem Druckreaktor realisieren läßt, kann die Geschwindigkeit des hydrolytischen Abbaus noch gesteigert werden. Chrom wird nach den Angaben dieser Patentschrift bis auf Restmengen "unterhalb von 5 ppm" entfernt, wobei jeodch die genauen Konzentrationen der Lösungen an Collagenhydrolysat und damit der Bezug für den Cr-Gehalt nicht offenbart ist.

Aus der deutschen Auslegeschrift DE 1 000 388 ist ein Verfahren zur Herstellung von Eiweiß-Abbauprodukten bekannt, bei der die Hydrolyse von Chromleder mit Wasser oder wäßrigem Ammoniak bei erhöhter Temperatur und erhöhtem Druck durchgeführt wird.

Der Wunsch nach einer Vermeidung der drastischen Reaktionsbedingungen der genannten klassischen Aufschlußverfahren von Proteinen hat in der Folge zur Entwicklungen enzymatischer Hydrolyse-Methoden geführt. Beispielsweise ist aus der deutschen Patentanmeldung DE 22 52 281 ein Verfahren zur Hydrolyse von Hautlappen mittels neutraler oder alkalischer Proteasen bekannt, die imstande sind, Kollagen zu zersetzen. Als Hautlappen können dabei unter anderem Lederlappen eingesetzt werden. Vor der enzymatischen Behandlung wird das Protein des Hautlappens durch Erhitzen mit Wasser denaturiert. Die enzymatische Hydrolyse wird anschließend bei moderaten Temperaturen im Bereich von 20 bis 70 °C durchgeführt. Als typische Aufarbeitungsschritte schließen sich die Hitzedenaturierung des Enzyms sowie die Filtration des erhaltenen Rohproduktes an.

In einer neueren Publikation haben Heideman et. al sich eingehend mit den Möglichkeiten der Aufarbeitung von Chromfalzspänen auseinandergesetzt. Bezüglich der Hydrolyse von Chromfalzspänen mit Proteasen kommen sie dabei zu dem Schluß, daß die vorangehende Denaturierung der Falzspäne eine entscheidende Rolle spielt. Sie erhielten dabei optimale Ergebnisse, wenn sie Chromfalzspäne zunächst ca. 20 Minuten mit 10 % Kalk aufschlossen und anschließend mit Protease weiterbehandelten (vergl.: Das Leder 1991, 42, 133-143).

Erst vor kurzem ist von Taylor et. al nachgewiesen worden, daß bei der enzymatischen Hydrolyse von Chromlederabfällen eine einleitende Denaturierung nicht zwingend notwendig ist. Nach ihrer Methode wird das in den Abfällen enthaltene Protein ohne vorherige Denaturierung bei moderaten Temperaturen mit speziellen Mischungen von Alkali- und Erdalkaliverbindungen und Alkalasen behandelt. Die Autoren haben über ihr Verfahren auf der 85. Jahrestagung der ALCA berichtet (J. Am. Leather Chem. Assoc. 1990, 85, 264); das Verfahren ist darüber hinaus Gegenstand des US-Patents 5,094,946. Darüber hinaus wurde die Wirksamkeit der Methode durch Praxisversuche der dänischen Gerberei Svendborg Fingarveri bestätigt ("Bio Times" [vierteljährlich erscheinendes Magazin der Fa. Novo Nordisk] 1990, 5, Nr.1, S.4-5). Die Methode von Taylor et al. hat jedoch den Nachteil, daß die so hergestellten wäßrigen Konzentrate nicht lagerstabil sind, sondern im Laufe der Zeit austrüben.

Aus DATA BASE WPI, Week 9127, Derwent Publications Ltd., London, GB (AN 91-209 003) und DD-A-287274 ist ein Verfahren zur Herstellung sulfatarmer Proteinhydrolysate auf enzymatischem Wege bekannt.

Gemäß DATA BASE WPI, Week 9131, Derwent Publications Ltd., London, GB (AN 91-225 297) und HU-A-55 797 lassen sich chromarme Proteinhydrolysate erhalten, indem man entsalzte chromgegerbte Leder mit überschüssigem Alkali behandelt und zwar derart, daß die Startkonzentration an OH-Ionen 0,03 - 3,3 mol/l beträgt und zum Schluß der Hydrolyse immer noch ein pH-Wert von etwa 12,6 bis 12,7 resultiert. Danach wird 3 bis 48 Stunden stehengelassen und erneut bei 1 bis 5 bar und 70 bis 150 °C hydrolysiert und zwar so lange, bis der Chromgehalt genügend abgesenkt ist (Dauer: bis zu 10 Stunden).

DE-C-972 090 betrifft ein Verfahren zur Herstellung von gut schäumenden Eiweißpräparaten für Nahrungsmittelzwecke durch hydrolytischem Abbau von pflanzlichen oder tierischen Eiweißstoffen durch alkalische Hydrolyse mit Calciumhydroxid.

### Beschreibung der Erfindung

Aufgabe der vorliegenden Erfindung war es daher, ein Verfahren zur Herstellung chromarmer Proteinhydrolysate zu entwickeln, das zu klaren und gegen Austrübungen stabilen Produkten führt. Dabei sollte zusätzlich die Aufgabe gelöst werden, ein Verfahren zur Herstellung von Proteinhydrolysaten mit - im Vergleich zum einschlägigen Stand der Technik - geringerem Chromgehalt zur Verfügung zu stellen, was insbesondere für die Verwendung dieser Produkte im Bereich von Kosmetik und Nahrungsmittelindustrie von Bedeutung ist.

Diese Aufgabe wurde erfindungsgemäß gelöst, indem man chromhaltige proteinhaltige Rohstoffe zunächst in wäßrigem Medium in Gegenwart eines Additivs aus der Gruppe der Erdalkalioxide oder -hydroxide sowie in Gegenwart proteolytischer Enzyme partiell hydrolysiert und die dabei erhaltenen Partialhydrolysate einer anschließenden chemischen Hydrolyse in Gegenwart von Erdalkalioxiden oder -hydroxiden bei einem pH-Wert im Bereich von 11 bis 13 unterwirft.

Gegenstand der vorliegenden Erfindung ist daher ein Verfahren zur Herstellung chromarmer Proteinhydrolysate durch Hydrolyse chromhaltiger proteinhaltiger Rohstoffe, wobei man die chromhaltigen proteinhaltigen Rohstoffe zunächst in wäßrigem Medium in Gegenwart eines Additivs aus der Gruppe der Erdalkalioxide oder -hydroxide und in Gegenwart proteolytischer Enzyme partiell hydrolysiert und die dabei erhaltenen Partialhydrolysate - gegebenenfalls nach vorheriger Abtrennung von den gebildeten unlöslichen Chromsalzen in an sich bekannter Weise - einer anschließenden chemischen Hydrolyse in Gegenwart von Alkaliverbindungen aus der Gruppe der Erdalkalioxide oder -hydroxide bei einem pH-Wert von 11 bis 13 unterwirft.

Das erfindungsgemäße Verfahren hat den Vorteil, daß die dabei erhaltenen Produkte auch in Form von Konzentraten mit einem Aktivsubstanzgehalt oberhalb von 40 Gew.-% gegen Austrübung bei Lagerung stabil sind und sich darüber hinaus durch einen außerordentlich niedrigen Chromgehalt auszeichnen.

Die Durchführung des ersten Schrittes des erfindungsgemäßen Verfahrens erfolgt in an sich bekannter Weise.

In einer bevorzugten Ausführungsform der vorliegenden Erfindung wird der erste Schritt, das heißt die partielle Hydrolyse in Gegenwart proteolytischer Enzyme, ohne vorherige Denaturierung des chromhaltigen proteinhaltigen Rohstoffs durchgeführt. Dabei wird im wesentlichen
a) der Rohstoff in wäßrigen Milieu vorgelegt,
b) ein Erdalkalioxid oder -hydroxid als Additiv zugefügt, um den gewünschten pH-Wert einzustellen und einen Co-Faktor für das Enzym bereitzustellen,
c) in Gegenwart eines proteolytischen Enzyms bei einer Temperatur und einem pH-Wert, die dem Temperaturoptimum bzw. pH-Optimum der verwendeten Protease entsprechen, umgesetzt. Typisch sind dabei Temperaturen von 60 bis 75 °C und pH-Werte von 10 bis 11.

In diesem Zusammenhang sei ausdrücklich auf die amerikanische Patentanmeldung US 5 094 946 verwiesen. Es kann gewünscht sein, das bei der enzymatischen Hydrolyse erhaltene Rohprodukt anschließend durch ein Trennverfahren wie Absitzenlassen, Zentrifugation oder Filtration in ein Partialhydrolysat und einen chromhaltigen Rückstand aufzutrennen.

Es hat sich gezeigt, daß für die Einstellung des pH-Wertes im ersten Verfahrensschritt aus verfahrensökonomischen Gründen insbesondere die Technik der pH-Statuierung in betracht kommt. Diese Technik besteht darin, dem bei der enzymatischen Hydrolyse durch dabei freigesetzte Protonen bedingten Abfall des pH-Wertes durch kontinuierliches Zudosieren von Basen entgegenzuwirken und den pH-Wert dadurch auf konstantem Niveau zu halten (vergleiche z. B. Jens Adler-Nissen, "Enzymatic Hydrolysis of Food Proteins" Elsevier, London 1986). Bei der Anwendung der pH-Statuierungs-Technik empfiehlt es sich, in der Nähe des pH-abhängigen Wirkungsoptimums des jeweils eingesetzten Enzyms zu arbeiten.

Für den zweiten Schritt des erfindungsgemäßen Verfahrens, die chemische Hydrolyse, ist wesentlich, daß er in Gegenwart von Verbindungen aus der Gruppe der Erdalkalioxide oder -hydroxide bei einem pH-Wert von 11 bis 13 durchgeführt wird. Niedrigere pH-Werte bewirken nur eine unvollständige Hydrolyse, so daß einerseits die Stabilität der Proteinhydrolysate gegen Austrübung und andererseits die gewünschten niedrigen Chromgehalte des wäßrigen Proteinhydrolysats nicht gewährleistet sind. Bei höheren pH-Werten besteht dagegen die Gefahr des Wiederauflösens des amphoteren Chrom(III)hydroxids (vergl. Hollemann-Wiberg, "Lehrbuch der anorganischen Chemie", 81-90. Auflage, Seite 876).

Als Rohstoffe können in dem erfindungsgemäßen Verfahren an sich alle chromhaltigen proteinhaltigen Stoffe eingesetzt werden. Bevorzugt sind dabei jedoch die in den Gerbereien im Zuge der Lederherstellung anfallenden Chromlederabfälle. Dabei sind aufgrund ihrer Teilchengröße im mm-Bereich Chromfalzspäne besonders bevorzugt. Entsprechend werden großflächigere Chromlederabfälle, beispielsweise Lederlappen, vor der Anwendung des erfindungsgemäßen Verfahrens zweckmäßigerweise auf mechanischem Wege zerkleinert.

Die sich an die enzymatische Hydrolyse anschließende chemische Hydrolyse wird vorzugsweise bei Temperaturen von 80 bis 100 °C, insbesondere 90 bis 95 °C, durchgeführt. Die Dauer der chemischen Hydrolyse sollte 5 Minuten nicht unterschreiten; als Obergrenze ist von einem Wert von 60 Minuten auszugehen. Als Base wird im Zuge der chemischen Hydrolyse bevorzugt Calciumhydroxid oder Calciumoxid eingesetzt.

Die Durchführung der chemischen Hydrolyse erfordert keine speziellen technologischen Maßnahmen. Sie kann daher in beliebigen Behältern mit Mischvorrichtung durchgeführt werden. Als Beispiel für einen geeigneten offenen Reaktor sei ein Gerbmischer genannt.

Im Anschluß an die chemische Hydrolyse wird das erhaltene Rohprodukt in an sich üblicher Weise aufgearbeitet. Die Aufarbeitung umfaßt dabei in der Regel
a) ein oder mehrere Filtrationsschritte, die im wesentlichen der Entfernung von gebildetem Chromhydroxid dienen. Die Filtration erfolgt dabei aus Viskositätsgründen in der Regel im Temperaturbereich von 60 bis 100 °C, insbesondere 90 bis 95 °C;
b) das Ausfällen von Calcium-Ionen, z. B. durch Zugabe von Oxalsäure und anschließende Abtrennung des gebildeten Calciumoxalats, z. B. durch Filtration oder durch Zusatz von Natriumcarbonat und Abtrennung des ausgefallenen Calciumcarbonates;
c) das Aufkonzentrieren des Proteinhydrolysates auf die gewünschte Konzentration.

Die nach dem erfindungsgemäßen Verfahren hergestellten Produkte eignen sich bevorzugt für die Verwendung in Kosmetika und Nahrungsmitteln.

Die folgenden Beispiele dienen der Erläuterung der Erfindung und sind nicht einschränkend zu verstehen.

### Beispiele

### 1. Allgemeines

In Tabelle 1 sind die erfindungsgemäßen Beispiele mit B1 bis B3, die Vergleiche mit V1 bis V2 gekennzeichnet.

### 2. Ausführungsbeispiele

### 2.1. Beschreibung des erfindungsgemäßen Verfahrens

### 2.1.1. Beispiel B1

### (a) Enzymatische Hydrolyse

300 g Chromfalzspäne wurden bei 60 °C in 900 g Wasser suspendiert. Anschließend wurde durch Zusatz von Calciumhydroxid der pH-Wert auf 9,5 bis 10,5 eingestellt. Es wurden 0,6 g einer handelsüblichen alkalischen Protease zugesetzt und die Mischung 1 Stunde bei 55 bis 60 °C gehalten. Während dieser Hydrolyse wurde der pH-Wert durch Zugabe von Calciumhydroxid im Bereich von 9,5 bis 10,5 gehalten.

### (b) Desaktivierung des Enzyms / Filtration

Anschließend wurde zur Desaktivierung des Enzyms auf 100 °C aufgeheizt und 15 Minuten bei dieser Temperatur gehalten. Zur Abtrennung von Chromhydroxid und eventuell ungelöstem Calciumhydroxid wurde bei 95 °C filtriert.

### (c) Chemische Hydrolyse

Durch Zufügen von weiterem Calciumhydroxid wurde der pH-Wert auf 11,0 angehoben. Anschließend wurde 30 Minuten auf 90 °C erhitzt, um die in der ersten Stufe erhaltenen höhermolekularen Proteinhydrolysat-Bruchstücke weiter zu hydrolysieren. Danach wurde ungelöstes Calciumhydroxid und ausgefälltes Chromoxid durch Filtration abgetrennt. Das so erhaltene Proteinhydrolysat wurde anschließend in dieser verdünnten Lösung mittels Atomabsorptions-Spektroskopie auf seinen Chromgehalt untersucht. Die ermittelten Werte - angegeben in µg/ml - können Tabelle 1 entnommen werden.

### (d) Weitere Aufarbeitung

Im Filtrat wurde gelöstes Calcium als Calcium-Oxalat ausgefällt und abfiltriert. Anschließend wurden die Proteinhydrolysat-Lösungen durch Abdestillieren von Wasser auf Konzentrationen von jeweils 40 und 50 Gew.-% eingestellt.

### 2.1.2. Beispiel B2

Beispiel B1 wurde wiederholt, wobei jedoch in Schritt (c) der pH-Wert durch Zufügen von Calciumhydroxid auf 11,5 eingestellt wurde.

### 2.1.3 Beispiel B3

Beispiel B1 wurde wiederholt, wobei jedoch in Schritt (c) der pH-Wert durch Zufügen von Calciumhydroxid auf 12,5 eingestellt wurde.

### 2.2. Beschreibung der Vergleichsversuche

### 2.2.1. Vergleichsbeispiel V1

Das unter 2.1.1. beschriebene Verfahren wurde wiederholt, wobei jedoch Schritt (c), d. h. die chemische Hydrolyse, weggelassen wurde.

### 2.2.2. Vergleichsbeispiel V2

Beispiel B1 wurde wiederholt, wobei jedoch im Schritt (c) der pH-Wert durch Zufügen von Calciumhydroxid auf 10,5 eingestellt wurde.

### 3. Auswertung der Versuche

Die erhaltenen Proteinhydrolysate (B1 bis B3 sowie V1 und V2) wurden jeweils bezüglich ihres Chromgehalts und ihres Lagerverhaltens analysiert. Die ermittelten Werte sind in Tabelle 1 zusammengestellt. Die Chromgehalte sind dabei in µg/ml ("ppm") zu verstehen; sie beziehen sich auf die wäßrige Produktmischung.

Wie aus Tabelle 1 hervorgeht, wiesen die nach dem erfindungsgemäßen Verfahren hergestellten Produkte (B1 bis B3) deutlich niedrigere Chromgehalte auf, als die Produkte der rein enzymatischen Hydrolyse (V1). Darüber hinaus blieben die nach dem erfindungsgemäßen Verfahren hergestellten Produkte auch bei Lagerung klar.

Daß der pH-Wert bei der chemischen Hydrolyse kritisch ist, geht klar aus Versuch V2 hervor, an dem erkennbar ist, daß zu niedrige pH-Werte einen signifikant höheren Chromgehalt verursachen.

**Tabelle 1**

| Versuch | chemische Hydrolyse | | | Chromgehalt^{a)} (µg/ml) | Aussehen des konzentrierten Produktes^{b)} | |
|---|---|---|---|---|---|---|
| | ja/nein | pH-Wert | Dauer | | sofort | nach 3 Tagen |
| B1 | ja | 11,0 | 30 min | < 0,2 | klar | klar |
| B2 | ja | 11,5 | 30 min | < 0,1^{c)} | klar | klar |
| B3 | ja | 12,5 | 30 min | < 0,1^{c)} | klar | klar |
| V1 | nein | - | - | 2,1 | klar | trüb |
| V2 | ja | 10,5 | 30 min | 0,8 | klar | klar |

| | | | | | | |
|---|---|---|---|---|---|---|
| ^{a)} bezogen auf die verdünnte wäßrige Produktmischung; Mittelwert aus jeweils drei Messungen | | | | | | |
| ^{b)} die Aussagen in dieser Spalte gelten gleichermaßen für Proteinhydrolysat-Konzentrate mit einem Aktivsubstanzgehalt von 40 Gew.-%, als auch von 50 Gew.-%. | | | | | | |
| ^{c)} die Nachweisgrenze lag bei der gewählten Analysemethode (Atomabsorptions-Spektroskopie) bei 0,1 µg/ml, d. h. die Angabe "<0,1" bedeutet, daß der Chromgehalt unterhalb der Nachweisgrenze lag. | | | | | | |

## Patentansprüche

1. Verfahren zur Herstellung chromarmer Proteinhydrolysate durch Hydrolyse chromhaltiger proteinhaltiger Rohstoffe, wobei man die chromhaltigen proteinhaltigen Rohstoffe zunächst in wäßrigem Medium in Gegenwart eines Additivs aus der Gruppe der Erdalkalioxide oder - hydroxide und in Gegenwart proteolytischer Enzyme partiell hydrolysiert, **dadurch gekennzeichnet,** daß man die Partialhydrolysate der enzymatischen Hydrolyse - gegebenenfalls nach vorheriger Abtrennung von den gebildeten unlöslichen Chromsalzen in an sich bekannter Weise - einer anschließenden chemischen Hydrolyse in Gegenwart von Alkaliverbindungen aus der Gruppe der Erdalkalioxide oder -hydroxide bei einem pH-Wert von 11 bis 13 unterwirft.

2. Verfahren nach Anspruch 1, wobei man die partielle enzymatische Hydrolyse ohne eine vorherige Denaturierung des chromhaltigen proteinhaltigen Rohstoffs durchführt.

3. Verfahren nach Anspruch 1 oder 2, wobei man als chromhaltige proteinhaltige Rohstoffe Chromiederabfälle, insbesondere Chromfalzspäne, einsetzt.

4. Verfahren nach einem der Ansprüche 1 bis 3, wobei man die bei der enzymatischen Hydrolyse anfallenden Partialhydrolysate vor der chemischen Hydrolyse bei Temparaturen von 80 bis 100 °C, insbesondere 90 bis 95 °C, zur Abtrennung von Chromhydroxid filtriert.

5. Verfahren nach einen der Ansprüche 1 bis 4, wobei man die chemische Hydrolyse bei Temperaturen von 80 bis 100 °C,insbesondere 90 bis 95 °C, durchführt.

6. Verfahren nach einem der Ansprüche 1 bis 5, wobei man die chemische Hydrolyse über einen Zeitraum von 5 bis 60 Minuten durchführt.

7. Verfahren nach einem der Ansprüche 1 bis 6, wobei man zur chemischen Hydrolyse als Alkaliverbindung Calciumhydroxid oder Calciumoxid einsetzt.

8. Verfahren nach einem der Ansprüche 1 bis 7, wobei man die Hydrolyseschritte in einem Gerbmischer durchführt.

9. Verwendung der nach einem der Ansprüche 1 bis 8 hergestellten Produkte in Kosmetika oder Nahrungsmitteln.

## Claims

1. A process for the production of low-chromium protein hydrolyzates by hydrolysis of chromium-containing protein-containing raw materials, the chromium-containing protein-containing raw materials first being partly hydrolyzed in aqueous medium in the presence of an additive from the group of alkaline earth metal oxides or hydroxides and in the presence of proteolytic enzymes, characterized in that the partial hydrolyzates of the enzymatic hydrolysis are subsequently subjected to chemical hydrolysis - optionally after preliminary separation from the insoluble chromium salts formed by methods known per se - in the presence of alkali compounds from the group of alkaline earth metal oxides or hydroxides at a pH value of 11 to 13.

2. A process as claimed in claim 1, characterized in that the partial enzymatic hydrolysis is carried out without preliminary denaturing of the chromium-containing protein-containing raw material.

3. A process as claimed in claim 1 or 2, characterized in that chrome leather waste, more particularly chrome shavings, is/are used as the chromium-containing protein-containing raw materials.

4. A process as claimed in any of claims 1 to 3, characterized in that, before chemical hydrolysis, the partial hydrolyzates obtained in the enzymatic hydrolysis are filtered at temperatures of 80 to 100°C and, more particularly, 90 to 95°C to remove chromium hydroxide.

5. A process as claimed in any of claims 1 to 4, characterized in that the chemical hydrolysis is carried out at temperatures of 80 to 100°C and, more particularly, at temperatures of 90 to 95°C.

6. A process as claimed in any of claims 1 to 5, characterized in that the chemical hydrolysis is carried out over a period of 5 to 60 minutes.

7. A process as claimed in any of claims 1 to 6, characterized in that calcium hydroxide or calcium oxide is used as the alkali compound for the chemical hydrolysis.

8. A process as claimed in any of claims 1 to 7, characterized in that the hydrolysis steps are carried out in a tanning mixer.

9. The use of the products produced by the process claimed in any of claims 1 to 8 in cosmetics or foods.

## Revendications

1. Procédé de préparation d'hydrolysats de protéine à faible teneur en chrome, par hydrolyse de matières premières renfermant des protéines contenant du chrome, dans lequel on hydrolyse partiellement en premier lieu en milieu aqueux en présence d'un additif choisi dans le groupe des oxydes ou des hydroxydes de métal alcalino-terreux et en présence d'enzymes protéolytiques,
caractérisé en ce que
l'on soumet l'hydrolysat partiel de l'hydrolyse enzymatique, le cas échéant après séparation préalable des sels de chrome insolubles formés d'une manière connue en soi, à une hydrolyse chimique subséquente en présence de dérivés alcalins choisis dans le groupe des oxydes et des hydroxydes de métal alcalino-terreux, à une valeur de pH allant de 11 à 13.

2. Procédé selon la revendication 1 dans lequel on effectue l'hydrolyse enzymatique sans dénaturation préalable de la matière première renfermant des protéines contenant du chrome.

3. Procédé selon la revendication 1 ou 2, dans lequel on met en oeuvre comme matière première renfermant des protéines contenant du chrome, des déchets de cuir au chrome, en particulier des rognures de peau au chrome.

4. Procédé selon l'une des revendications 1 à 3, dans lequel les hydrolysats partiels qui se forment au cours de l'hydrolyse enzymatique, sont filtrés avant l'hydrolyse chimique à des températures de 80 à 100°C, en particulier de 90 à 95°C, en vue de la séparation de l'hydroxyde de chrome.

5. Procédé selon l'une des revendications 1 à 4, dans lequel on effectue l'hydrolyse chimique à des températures allant de 80 à 100°C, en particulier de 90 à 95°C.

6. Procédé selon l'une des revendications 1 à 5, dans lequel on effectue l'hydrolyse chimique pendant un espace de temps de 5 à 60 minutes.

7. Procédé selon l'une des revendications 1 à 6, dans lequel on met en oeuvre pour l'hydrolyse chimique comme composé alcalin, de l'hydroxyde de calcium ou de l'oxyde de calcium.

8. Procédé selon l'une des revendications 1 à 7, dans lequel on effectue les étapes d'hydrolyse dans un mélangeur de tannage.

9. Utilisation des produits préparés selon l'une des revendications 1 à 8, dans des cosmétiques ou dans des aliments.
